# EUROPEAN PATENT SPECIFICATION

(11) **EP 3 511 048 B1**
(45) Date of publication and mention of the grant of the patent: **27.11.2024**
(21) Application number: 17848983.7
(22) Date of filing: 31.07.2017
(51) Int. Cl.: A61M 39/22, A61H 15/00, A61H 15/02, A61M 37/00

(54) **NEEDLE DISC ROLLER APPARATUS**
NADELSCHEIBENWALZVORRICHTUNG
APPAREIL DE ROULEAU DE DISQUE À AIGUILLES

(30) Priority: 12.09.2016 KR 20160117166; 12.09.2016 KR 20160117175; 12.09.2016 KR 20160117193
(43) Date of publication of application: 17.07.2019
(73) Proprietor: K.L Global Co., Ltd., Seoul 04784 (KR)
(72) Inventor: LEE, Changwoo, Namyangiu-si, Gyeonggi-do 12257 (KR)
(74) Representative: dompatent von Kreisler Selting Werner - Partnerschaft von Patent- und Rechtsanwälten mbB
(86) International application number: PCT/KR2017/008225
(87) International publication number: WO 2018/048099

(56) References cited:
- CN-A- 101 670 145
- KR-A- 20110 103 111
- KR-A- 20110 131 983
- KR-A- 20140 044 098
- KR-B1- 100 873 221
- KR-B1- 101 573 252
- KR-B1- 101 573 252
- KR-B1- 101 647 009
- KR-B1- 101 682 863
- KR-B1- 101 700 563
- KR-B1- 101 701 097
- US-A1- 2014 343 591

## Description

### [Technical Field]

The following disclosure relates to a needle disc roller apparatus for forming fine needle holes in skin using a needle disc roller and for injecting a drug through the needle holes, and more particularly, to a coaxial double rotation type needle disc roller apparatus which enables more efficient needling procedure by rotating a needle disc roller while contacting needle discs provided in the needle disc roller with the skin and by doubly rotating by a core rotation shaft and a hollow rotation shaft that are coaxial to each other while restraining the needle discs in the needle disc roller with respect to each other.

### [Background Art]

There has been proposed a needle roller apparatus in which a head of a roller is coupled to the upper end of a drug container filled with a drug, and a hole is formed between the drug container and the head to allow the drug to come out. In this needle roller apparatus, an opening formed at the upper end of the drug container is formed as an elastically deformable incision portion, and has a valve structure in which the incision portion is pushed by the weight of a ball located in the inside thereof when the drug container is inverted. However, due to the delivery method in which the drug falls on a remote needle disc, this apparatus is difficult to reliably deliver a drug to each of the needles, and it is difficult to control the deformation of the incision portion due to the ball weight. Moreover, since the incision portion is permanently deformed as the period of use increases, the apparatus may become unusable. Furthermore, the related art has inconvenience in coupling the needle disc roller to the drug container. In addition, the related-art apparatus is not good in assembling property in that assembling of the assembly including the needle disc roller requires bolt fastening, adhesion, and the like.

Particularly, in the related art, when the needle disc roller is pressed and rotatably operated on the skin in such a manner that the entire needle disc roller is rotated with respect to one shaft, the smoothness of rotation is reduced. In addition, when rotation fails, it is apprehended that the needle disc will damage the skin due to an external force that pushes the needle disc roller against the skin.

KR 101 573 252 B1, KR 101 647009 B1, KR 100 873 221 B1 and CN 101 670 145 B disclose a needle disc roller apparatus with the features of the preamble of claim 1.

### [Disclosure]

### [Technical Problem]

It has been conventionally considered that a plurality of needle discs are simply rotatably mounted on one shaft such that the needle discs are individually rotated. However, in this case, during the procedure using the needle disc roller, there is a high possibility that some needle discs rotate more and cause an undesired deviation among the needle discs, which causes reliable operation to be difficult. Although it has been considered to use one integral needle roller including several needle disc shapes instead of using a plurality of needle discs, this makes it difficult to precisely form a needle, and thus makes it difficult to put to practical use.

Accordingly, the present disclosure provides a coaxial double rotation type needle disc roller apparatus which enables more efficient needling procedure by rotating a needle disc roller while contacting needle discs provided in a needle disc roller with the skin and by doubly rotating by a core rotation shaft and a hollow rotation shaft that are coaxial to each other while restraining the needle discs spaced by a plurality of spacers in the needle disc roller with respect to each other.

The present disclosure also provides a needle disc roller apparatus in which a valve hole formed in a valve membrane is elastically expanded by retraction of a needle disc roller when the needle disc roller is pressed against the skin, allowing a drug of a drug container to be reliably delivered to the needle disc of the needle disc roller. Also, since there is no incision around the valve hole, there is almost no permanent deformation, and the amount of drug delivery can be appropriately controlled.

The present disclosure also provides a needle disc roller apparatus which includes a bidirectional connection structure is capable of reliable one-touch sealing connection to a connection portion of a drug container, and capable of reliable sealing connection with a roller guiding holder of the needle disc roller apparatus.

### [Technical Solution]

The invention is defined by the features of claim 1. According to the invention, a needle disc roller apparatus includes: a connection cap (12); a roller guiding holder (14) coupled to a front end of the connection cap (12); and a needle disc roller (20) maintained so as to be movable forward and backward in the roller guiding holder (14), wherein the needle disc roller (20) includes: a roller bracket (22) installed to be rectilinearly movable forward and backward along the guide groove (142); a core rotation shaft (21) rotatably installed for the roller bracket (22), a roller bobbin (24) rotatably installed for the core rotation shaft (21); and a plurality of ring-shaped needle discs (26) and a plurality of ring-shaped spacers (28) that are all restrained by the roller bobbin (24) and rotate about the core rotation shaft (21) together with the roller bobbin (24), the roller bracket (22) includes: a base portion (221); and a pair of shaft mounting walls (222 and 222) vertically formed on both sides of the front surface of the base portion (221), and the pair of shaft mounting walls (222 has shaft holes (2224 and 2224) in which the core rotation shaft (21) is rotatably fitted, and each of the pair of shaft mounting walls (222) is slidably fitted in the guide groove (142). The needle disc roller further includes a drug delivery mechanism for delivering a drug in a drug container to ring-shaped needle discs (26) of the needle disc roller (20) in response to the movement of the needle disc roller (20) back and forth as the needle disc roller (20) is pressed against the skin.

Here, the roller bobbin (24) may include: a hollow rotation shaft (241) rotatably installed on the outer circumferential surface of the core rotation shaft (21) so as to be rotatable about the core rotation shaft (21), and; a pair of bobbin covers (242a and 242b) provided at both ends of the hollow rotation shaft (241) to prevent separation of the plurality of ring-shaped needle discs (26) and the plurality of ring-shaped spacers (28). At least one bobbin cover (242b) of the pair of bobbin covers (242a and 242b) may be detachably assembled with respect to the hollow rotation shaft (241). At least one key groove (2412) may be formed on the outer circumference of the hollow rotation shaft (241) in the longitudinal direction, each of the plurality of ring-shaped needle discs (26) may include a first key (265) on an inner surface thereof to be fitted in the key groove (2412), each of the plurality of ring-shaped spacers may include a second key on an inner circumferential surface thereof to be fitted in the key groove (2412), and the plurality of ring-shaped needle discs (26) and the plurality of ring-shaped spacers (28) may be restrained by the hollow rotation shaft (241) by fitting the first key (265) and the second key (285) of the plurality of ring-shaped needle discs (26) and the plurality of ring-shaped spacers (28) into the key groove (2412) in a row. The drug delivery mechanism may include: a valve membrane (123) of a viscoelastic material having a valve hole (1232) which is formed in the connection cap (12) and communicates with a front end opening of the drug container; and a valve operating part (23) selectively opening and closing the valve hole (1232) by moving forward and backward together with the needle disc roller (20), and the valve operating part (23) includes: a rod portion (231) moving forward and backward integrally with the needle disc roller (20) while being inserted into the valve hole (1232) without a gap; a tapered pressing portion (232) formed in the middle of the rod portion (231) and elastically expanding the valve hole (1232) by being fitted into the valve hole (1232) when moving backward together with the rod portion (231); and at least one drug passage incision groove (233) formed on the tapered pressing portion (232) and connecting to the valve hole (1232) to open the valve hole (1232) when the valve hole (1232) is expanded by the tapered pressing portion (232).

In another general aspect, a needle disc roller apparatus includes: a viscoelastic connection cap (12) coupled to cover a front end opening of a drug container (2); a roller guiding holder (14) coupled to a front end of the viscoelastic connection cap (12); a needle disc roller (20) maintained so as to be movable forward and backward in the roller guiding holder (14); and a drug delivery mechanism delivering a drug in a drug container (2) to a needle disc (26) of the needle disc roller (20) when pressing the needle disc roller (20) on the skin, wherein the drug delivery mechanism includes: a valve membrane (123) of a viscoelastic material having a valve hole (1232) which is formed in the viscoelastic connection cap (12) and communicates with a front end opening of the drug container; and a valve operating part (23) selectively opening and closing the valve hole (1232) by moving forward and backward together with the needle disc roller (20), and the valve operating part (23) includes: a rod portion (231) moving forward and backward integrally with the needle disc roller (20) while being inserted into the valve hole (1232) without a gap; a tapered pressing portion (232) formed in the middle of the rod portion (231) and elastically expanding the valve hole (1232) by being fitted into the valve hole (1232) when moving backward together with the rod portion (231); and at least one drug passage incision groove (233) formed on the tapered pressing portion (232) and connecting to the valve hole (1232) to open the valve hole (1232) when the valve hole (1232) is expanded by the tapered pressing portion (232).

Here, the viscoelastic connection cap (12) may include: a rear outer tube (120a) closely adhered to the outer circumferential surface of the front end opening of the drug container (2) when being coupled to the front end of the drug container (2); a rear outer tube (120b) inserted into an opening and closely adhered to the inner circumferential surface of the opening thereof; a plate-shaped ring (120c) connecting the inner circumference of the front end of the rear outer tube (120a) and the outer circumference of the front end of the rear inner tube (120b); a front outer tube (121) closely adhered to the outer surface of a portion of a rear end of the roller guiding holder (14) when being coupled to the rear end of the roller guiding holder (14); and a front inner tube (122) having a concentric axis and formed inside the front outer tube (121). The valve membrane (123) may be integrally formed inside the hollow of the front inner tube (122), and the valve hole (1232) may be formed at the center of the valve membrane (123). The tapered pressing portion (232) may have a shape gradually increasing in diameter from the rear end having the same diameter as the rod portion (231) toward the front end having a diameter larger than the diameter of the rod portion (231). The drug passage incision groove (233) may form a pair with the drug passage incision groove (233) formed at one side of the tapered pressing portion (232) while being formed at the other side of the tapered pressing portion (232), and the pair of drug passage incision grooves (233 and 233) may be formed adjacent to the outer circumference of the needle disc (26) and opposite to each other.

In another general aspect, a needle disc roller apparatus may include: a viscoelastic connection cap (12) coupled to cover a front end opening of a drug container (2); a roller guiding holder (14) coupled to a front end of the viscoelastic connection cap (12); a needle disc roller (20) maintained so as to be movable forward and backward in the roller guiding holder (14); and a drug delivery mechanism delivering a drug in a drug container (2) to a needle disc (26) of the needle disc roller (20) when pressing the needle disc roller (20) on the skin, wherein the viscoelastic connection cap (12) includes: a rear outer tube (120a) having a circular inner circumference that is closely adhered to the outer circumferential surface of the front end opening of the drug container (2) when being coupled to the front end of the drug container (2); a rear outer tube (120b) having a circular outer circumference that is inserted into the opening of the drug container (2) and is closely adhered to the inner circumferential surface of the opening thereof; a plate-shaped ring (120c) connecting the inner circumference of the front end of the rear outer tube (120a) and the outer circumference of the front end of the rear inner tube (120b); a front outer tube (121) closely adhered to the outer surface of a rear end step (140) of the roller guiding holder (14) when being coupled to the rear end of the roller guiding holder (14); a front inner tube (128) having a concentric axis and formed inside the front outer tube (121); a hook (122) formed so as to direct to the inside from the rear end of the rear outer tube (120a) and thus hooked at the stopping protrusion (2a) of the drug container (2) in a one-touch manner when being assembled with the viscoelastic connection cap (12) and the drug container (2); and a ring-shaped retaining cover (121a) extending inward from the front end of the front outer casing (121) and elastically deformed such that the rear end step (140) of the roller guiding holder (14) is inserted into the inside thereof.

Here, a valve membrane (123) may be integrally formed inside the hollow of the front inner tube part (128), the valve membrane (123) may be a valve hole (1232) at the center thereof, and the valve hole (1232) may be elastically expanded or contracted by an external force. The drug delivery mechanism may include a valve operating part (23) provided on the needle disc roller (20) together with the valve membrane (123), and the valve operating part (23) may interact with the valve membrane (123) in accordance with the forward and backward movement of the valve disc (12) to open or close the valve hole (1232). The valve operating part may include: a rod portion moving forward and backward integrally with the needle disc roller (20) while being fitted in the valve hole without a gap; a tapered pressing portion (234) formed at the center of the rod portion (231) and having a shape gradually increasing in diameter from the rear end having the same diameter as the rod portion (231) toward the front end having a diameter larger than the diameter of the rod portion (231); and a drug passage incision groove (233) formed in the tapered pressing portion (232), wherein when the rod portion (231) moves backward and the tapered pressing portion (232) presses the valve membrane (123), the valve membrane (123) is pushed by the tapered pressing portion (232) to expand the valve hole (1232), and the valve hole (1232) is connected to the drug passage incision groove (233) and is opened while being expanded by the tapered pressing portion (232).

Other features and aspects will be apparent from the following detailed description, the drawings, and the claims.

### [Advantageous Effects]

A coaxial double rotation type needle disc roller apparatus according to an embodiment of the present invention enables more efficient needling procedure, when rotatably operating a needle disc roller while contacting needle discs provided in a needle disc roller with the skin, by doubly rotating the needle discs by a core rotation shaft and a hollow rotation shaft that are coaxial to each other while restraining the needle discs spaced by a plurality of spacers in the needle disc roller with respect to each other.

A needle disc roller apparatus according to an embodiment of the present invention can reliably deliver a drug of a drug container through a flow path by securing the flow path connected to a valve hole while the valve hole formed in a valve membrane is being elastically expanded by retraction of a needle disc roller when the needle disc roller is pressed against the skin. In this case, since there is no incision around the valve hole, there is almost no permanent deformation, and the amount of drug delivery can be appropriately controlled.

### [Description of Drawings]

FIG. 1 is a perspective view illustrating a needle disc roller apparatus according to an embodiment of the present invention.
FIGS. 2A and 2B are exploded perspective views illustrating a needle disc roller apparatus according to an embodiment of the present invention.
FIG. 3 is a cross-sectional view illustrating the needle disc roller apparatus shown in FIGS. 1, 2A and 2B.
FIG. 4 is a perspective view illustrating a needle disc roller.
FIG. 5 is an exploded perspective view illustrating a needle disc roller.
FIG. 6 is a view illustrating an action of a drug delivery mechanism of a needle disc roller apparatus according to an embodiment of the present invention.

### [Best Mode]

Hereinafter, preferred embodiments of the present invention will be described in detail with reference to the accompanying drawings. The accompanying drawings and the description thereof are intended to help understanding of the present invention for those of ordinary skill in the art. Accordingly, the drawings and description are not to be construed as limiting the scope of the present invention.

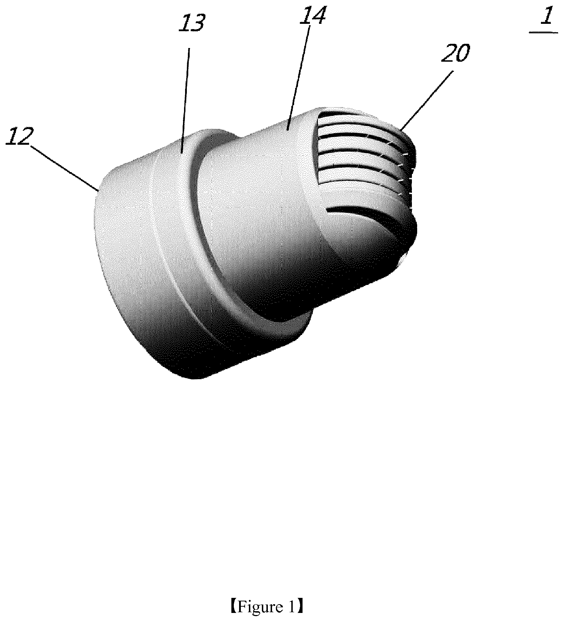

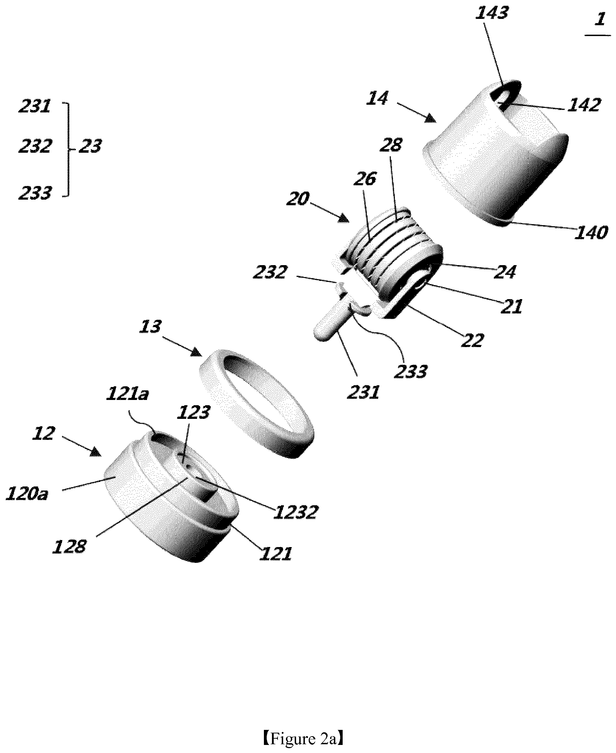

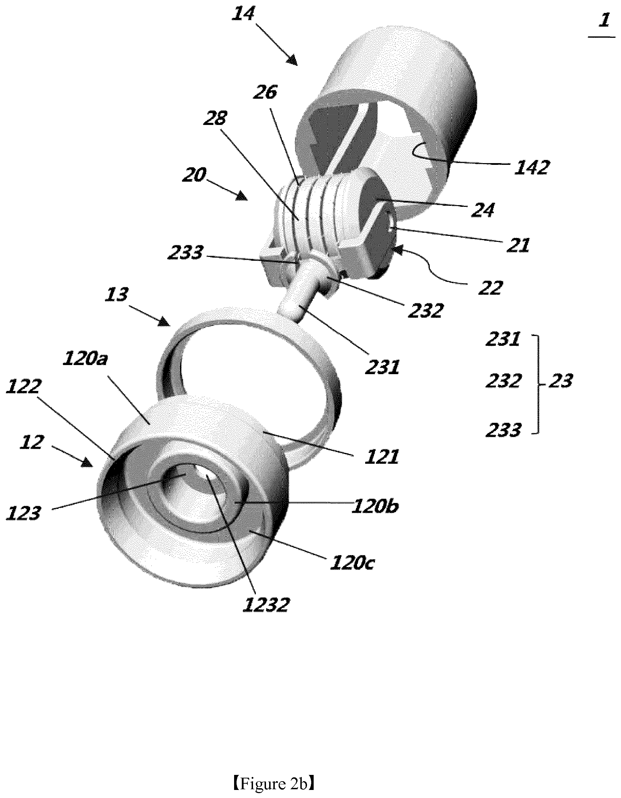

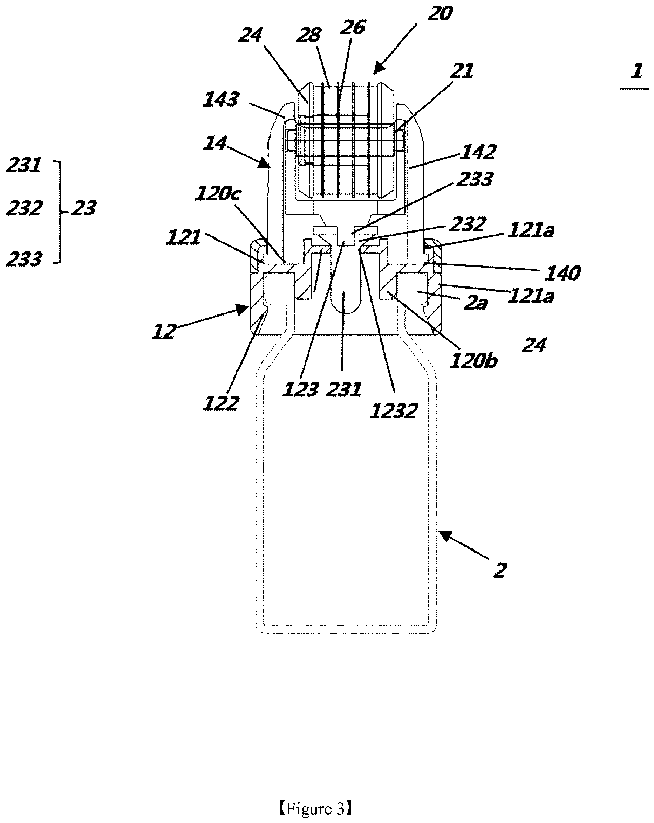

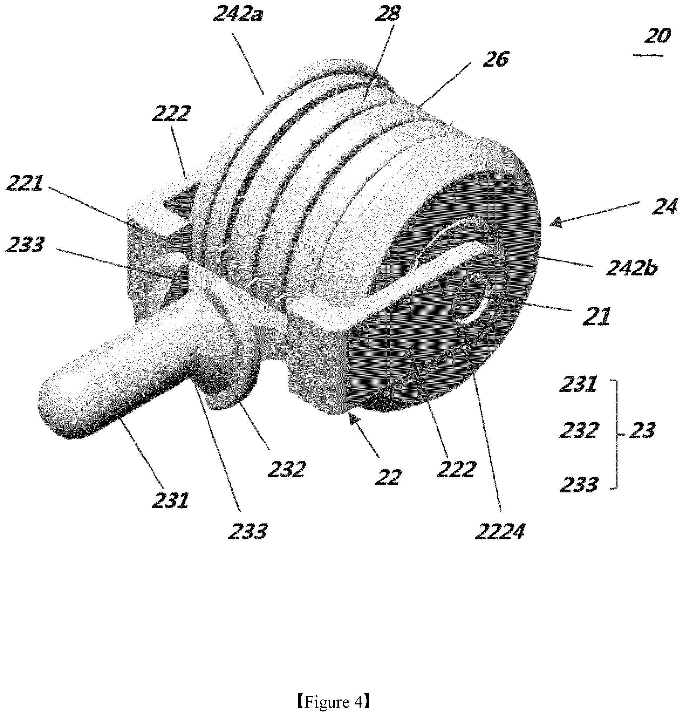

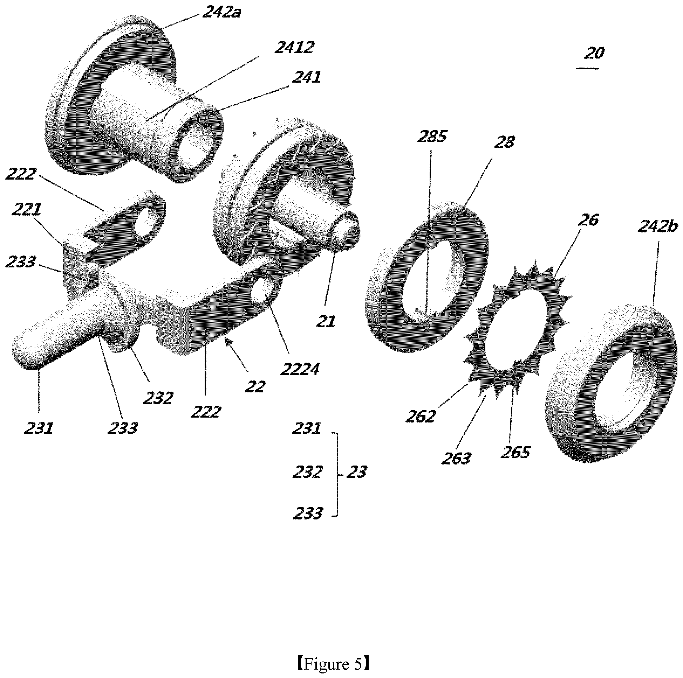

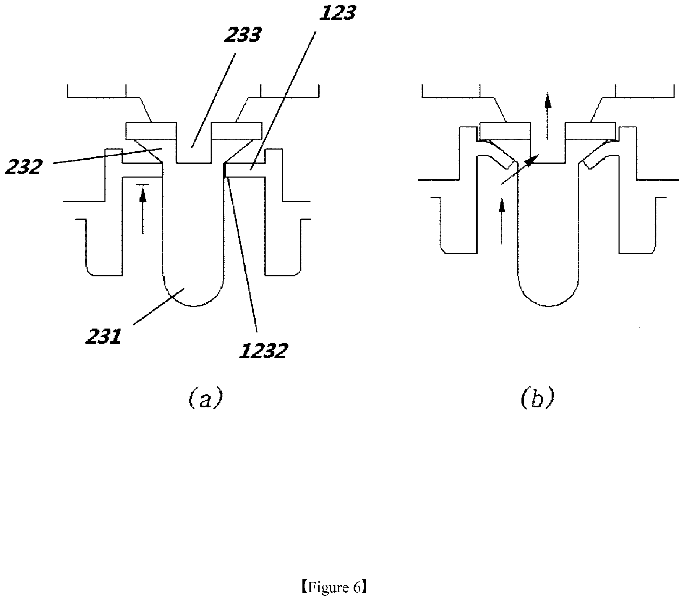

FIG. 1 is a perspective view illustrating a needle disc roller apparatus according to an embodiment of the present invention. FIGS. 2A and 2B are exploded perspective views illustrating a needle disc roller apparatus according to an embodiment of the present invention. FIG. 3 is a cross-sectional view illustrating the needle disc roller apparatus shown in FIGS. 1, 2A and 2B. FIG. 4 is a perspective view illustrating a needle disc roller. FIG. 5 is an exploded perspective view illustrating a needle disc roller. FIG. 6 is a view illustrating an action of a drug delivery mechanism of a needle disc roller apparatus according to an embodiment of the present invention.

As shown in FIGS. 1 to 6, a needle disc roller apparatus 1 according to an embodiment of the present invention is used in combination with the front end of a drug container 2 having an opening. Also, the needle disc roller apparatus 1 includes a viscoelastic connection cap 12 coupled to cover the front end opening of the drug container 2, a roller guiding holder 14 coupled to the front end of the viscoelastic connection cap 12, a needle disc roller 20 maintained so as to be movable forward and backward in the roller guiding holder 14, and a drug delivery mechanism for delivering a drug in the drug container 2 to the needle disc 26 of the needle disc roller 20 when pressurizing the needle disc roller 20 against the skin.

The needle disc roller apparatus 1 may further include a connection ring 13 for a more reliable assembly between the viscoelastic connection cap 12 and the roller guiding holder 14.

In the present embodiment, the drug container 2 stores a drug in a general form of medicine bottle. This drug may be various kinds of drugs that may help the needling procedure of punching a plurality of fine holes in the skin. In addition, the drug container 2 includes a ring-shaped stopping protrusion 2a protruding in a radial direction on the outer circumferential surface of the front end opening. The stopping protrusion 2a is used for one-touch coupling with the viscoelastic connection cap 12, which will be described in detail below.

The viscoelastic connection cap 12, as a part of the needle disc roller apparatus 1, allows the needle disc roller apparatus 1 to be reliably assembled to the drug container 2 in a sealed state. Also, the viscoelastic connection cap 12 is configured to be detachably coupled to the drug container 2 in a one-touch manner, and to be detachably coupled to the roller guiding holder 14.

The viscoelastic connection cap 12 is formed of a silicone material that is a viscoelastic material capable of enhancing the assembling property with the drug container 2 and the assembling property with the roller guiding holder 14, and capable of improving the elastic expandability and contractibility of the valve hole which will be described in detail below.

The viscoelastic connection cap 12 includes a rear outer tube 120a having a circular inner circumference that is closely adhered to the outer circumferential surface of the front end opening of the drug container 2 when being coupled to the front end of the drug container 2, a rear outer tube 120b having a circular outer circumference that is inserted into the opening of the drug container 2 and is closely adhered to the inner circumferential surface of the opening thereof, a plate-shaped ring 120c connecting the inner circumference of the front end of the rear outer tube 120a and the outer circumference of the front end of the rear inner tube 120b, a front outer tube 121 closely adhered to the outer surface of a rear end step 140 of the roller guiding holder 14 when being coupled to the rear end of the roller guiding holder 14, and a front inner tube 128 having a concentric axis and formed inside the front outer tube 121.

The viscoelastic connection cap 12 integrally includes a hook 122 that is formed so as to direct to the inside from the rear end of the rear outer tube 120a and thus hooked at the stopping protrusion 2a of the drug container 2 in a one-touch manner when being assembled with the viscoelastic connection cap 12 and the drug container 2. In the process of coupling the viscoelastic connection cap 12 coupled to the roller guiding holder 14 maintaining the needle disc roller 20 to be movable forward and backward to the front end of the drug container 2, the outer tube 120a of the drug container 2 is expanded and elastically deformed in a radial direction. When the coupling is completed, the outer tube 120a is restored to its original state, and the hook 122 is hooked at the stopping protrusion 2a of the drug container 2. Thus, one-touch fixing of the viscoelastic connection cap 14 is completed.

The viscoelastic connection cap 12 includes a ring-shaped retaining cover 121a extending inward from the front end of the front outer casing 121. The ring-shaped retaining cover 121a, as a part of the viscoelastic connection cap 12, has viscoelasticity, and thus is elastically deformed such that the rear end step 140 of the roller guiding holder 14 is inserted into the inside thereof. On the other hand, when the rear end step 140 is inserted, the ring-shaped retaining cover 121a is closely adhered to the front surface of the rear end step 140 and tightly fixes the roller guiding holder 14 to the viscoelastic connection cap 12 in a sealed state.

In addition, the viscoelastic connection cap 12 integrally includes a valve membrane 123 integrally formed in the inside of the hollow of the front inner tube 128. The valve membrane 123 has a valve hole 1232 at the center thereof, and allows the valve hole 1232 to be elastically expanded or contracted by an external force due to the characteristics of a viscoelastic material, especially, silicone material. The valve membrane 123 with the valve hole 1232 constitutes one drug delivery mechanism together with a valve operating part 23 provided on the needle disc roller 20. The valve operating part 23 opens and closes the valve hole 1232 by interacting with the valve membrane 123 as the needle disc roller 20 moves forward or backward.

The rear inner tube 120b of the viscoelastic connection cap 12 is formed to have a diameter such that the entire outer circumference thereof can be closely inserted into the opening of the drug container 2 without any gap. Accordingly, when the viscoelastic connection cap 12 is coupled to the drug container 2, the rear inner tube 120 makes complete contact with the inner circumferential surface of the opening of the drug container 2 to prevent a drug from leaking.

The roller guiding holder 14 is a hollow cylindrical structure having front and rear sides opened. The roller guiding holder 14 allows the needle disc roller 20 to be movable forward and backward when the needle disc roller 20 is fitted in the hollow of the roller guiding holder 14. The roller guiding holder 14 is provided with a pair of guide grooves 142 guiding the forward and backward linear motion of the needle disc roller 20. The pair of guide grooves 142 is provided so as to face each other on the inner circumferential surface of the roller guiding holder 14. The needle guiding holder 14 includes a front end stopper 143 to prevent the needle guide roller 20 from separating from the front end of the guide groove 142.

The needle disc roller 20 is held by the roller guiding holder 14 so as to be linearly movable backward and forward. The needle disc roller 20 includes a roller bracket 22 installed to be rectilinearly movable forward and backward along the guide groove 142, a core rotation shaft 21 rotatably installed for the roller bracket 22, a roller bobbin 24 rotatably installed for the core rotation shaft 21, and a plurality of ring-shaped needle discs 26 and a plurality of ring-shaped spacers 28 that are all restrained by the roller bobbin 24 and rotate about the core rotation shaft 21 together with the roller bobbin 24.

As shown in FIG. 5, the roller bobbin 24 includes a hollow rotation shaft 241 rotatably installed on the outer circumferential surface of the core rotation shaft 21 so as to be rotatable about the core rotation shaft 21, and a pair of bobbin covers 242a and 242b provided at both ends of the hollow rotation shaft 241 to prevent separation of a disc-spacer set including the plurality of ring-shaped needle discs 26 and the plurality of ring-shaped spacers 28. In this case, at least one bobbin cover 242b of the pair of bobbin covers 242a and 242b may be detachably assembled to the hollow rotation shaft 241 so as to facilitate assembly and separation of the ring-shaped needle discs 26 and ring-shaped spacers 28.

Thus, the plurality of ring-shaped needle discs 26 and the plurality of ring-shaped spacers 28 may be restrained by the hollow rotation shaft 241 and may be integrally rotated although the plurality of ring-shaped needle discs 26 and the plurality of ring-shaped spacers 28 are assembled in a separated state. As a unit for restraining the ring-shaped needle discs 26 and the ring-shaped spacers 26, a key groove 2412 formed on the outer circumferential surface of the hollow rotation shaft 241 in a longitudinal direction, and first keys 265 formed on the inner circumferential surfaces of the respective needle discs 26 and the second keys 285 formed on the inner circumferential surfaces of the respective spacers 28 are provided.

Each of the plurality of needle discs 26 includes a plurality of sawtooth-shaped needles 262 at a certain interval along the outer circumference thereof. Also, a drug delivery groove 263 is formed between neighboring needles 262.

The pair of bobbin covers 242a and 242b includes a first bobbin cover 242a integrated with the hollow shaft 241 and a second bobbin cover 242b assembled with the first bobbin cover 242a. When the bobbin cover 242b and the hollow shaft 241 are disassembled, the ring-shaped needle discs 26 and the ring-shaped spacers 28 may be mounted onto or separated from the outer circumferential surface of the hollow rotation shaft 241. The ring-shaped needle discs 26 and the ring-shaped spacers 28 are alternately arranged on the outer circumferential surface of the hollow rotation shaft 241. In this case, the rectangular first keys 265 formed on the ring-shaped needle discs 26 and the rectangular second keys 285 formed on the ring-shaped spacers 28 may be fitted in the key groove 2412 formed on the outer circumferential surface of the hollow rotation shaft 241 in a row. Thus, the ring-shaped needle discs 26 and the ring-shaped spacers 28 may be restrained by the hollow rotation shaft 26 and may be integrally rotated. Although a failure occurs in some of the plurality of needle discs during the assembly process of the needle disc roller 20, the needle disc roller 20 may be manufactured by replacing only the corresponding needle discs.

On the other hand, the roller bracket 22 includes a base portion 221 and a pair of shaft mounting walls 222 and 222 vertically formed on both sides of the front surface of the base portion 221. Also, shaft holes 2224 and 2224 in which the core rotation shaft 21 is rotatably fitted are formed in the pair of shaft mounting walls 222. Each of the pair of shaft mounting walls 222 is slidably fitted in the guide groove 142 (see FIGS. 1 to 3) of the roller guiding holder 14 described above.

Referring to FIGS. 2A, 2B, 3, 4 and 5, the valve operating part 23 constituting a part of the drug delivery mechanism is formed on the rear surface of the base portion 221. The valve operating part 23 includes a rod portion 231 perpendicular to the base portion 221 and longitudinally extended from the center of the rear surface of the base portion 221, and a tapered pressing portion 232 formed on the outer circumferential surface of the rod portion 231. The rod portion 231 is fitted into the circular valve hole 1232 formed in the valve membrane 123 in a sealed state without forming a fine gap, and moves forward and backward together with the forward and backward movement of the needle disc roller 20. The tapered pressing portion 232 is formed in the middle of the rod portion 231, and has a shape gradually increasing in diameter from the rear end having the same diameter as the rod portion 231 toward the front end having a diameter larger than the diameter of the rod portion 231. When the rod portion 231 moves backward and thus the tapered pressing portion 232 presses the valve membrane 123, the size of the valve hole 1232 formed in the valve membrane 123 is expanded in accordance with the increase of the diameter of the tapered pressing portion 232. On the other hand, a drug passage incision groove 233 is formed from the outer side of the tapered pressing portion 232 having a largest diameter to the inner side of the tapered pressing portion 232 having a smallest diameter. The drug passage incision groove 233 allows a drug to pass therethrough while the tapered pressing portion 232 is pressing the valve hole 1232. When the drug passage incision groove 233 is not formed in the tapered pressing portion 232, even if the tapered pressing portion 232 presses the valve membrane 123 to expand the valve hole 1232, the valve hole 123 is blocked by the tapered pressing portion 232, resulting in failure of passage of the drug. In this embodiment, a part of the valve hole 1232 expanded is connected to the drug passage incision groove 233 to allow the drug to pass therethrough. The tapered pressing portion 232 has a pair of drug passage incision grooves 233 most adjacently to the outer circumference of the needle disc-spacer set, and the pair of drug passage incision grooves 233 are formed at positions opposed to each other so as to smoothly supply the drug regardless of the clockwise or counterclockwise direction of the needle disc 26. More specifically, the pair of drug passage incision grooves 233 is formed in a form of bisecting the tapered pressing portion 232 in parallel with the needle disc 26.

FIG. 6 is a view illustrating an action of a drug delivery mechanism of a needle disc roller apparatus according to an embodiment of the present invention. Referring to 6A, when the needle disc roller is not pressed against the skin, the rod portion 231 is inserted into the valve hole 1232 formed in the valve membrane 123 in a sealed state to prevent from leaking from the drug container. In this case, since the rod portion 231 and the tapered pressing portion 232 formed on the rod portion 231 do not move backward, the valve membrane 123 is not pressurized. Accordingly, the valve hole 1232 formed in the valve membrane 123 is not elastically expanded.

When the needle disc roller is pressed against the skin, the rod portion 231 and the tapered pressing portion 232 moves backward as shown in FIG. 6B to push back and deform the valve membrane 123 and simultaneously expand the valve hole 1232. Since the tapered pressing portion 232 is fitted into the valve hole 1232 while expanding the valve hole 1232, the valve hole 1232 is opened through the drug passage incision groove 233 formed on the tapered pressing portion 232. Accordingly, a drug stored in the drug container is supplied from the drug container to the needle disc of the disc roller through the valve hole 1232 and the drug passage incision groove 233.

A number of exemplary embodiments have been described above. Nevertheless, it will be understood that various modifications can be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

## Claims

1. A needle disc roller apparatus comprising:
a connection cap (12);
a roller guiding holder (14) coupled to a front end of the connection cap (12); and
a needle disc roller (20),
wherein the needle disc roller (20) comprises:
a roller bracket (22);
a core rotation shaft (21) rotatably installed for the roller bracket (22), a roller bobbin (24) rotatably installed for the core rotation shaft (21); and
a plurality of ring-shaped needle discs (26) and a plurality of ring-shaped spacers (28) that are all restrained by the roller bobbin (24) and rotate about the core rotation shaft (21) together with the roller bobbin (24),
the roller bracket (22) comprises:
a base portion (221); and
a pair of shaft mounting walls (222 and 222) vertically formed on both sides of the front surface of the base portion (221), and
the pair of shaft mounting walls (222) has shaft holes (2224 and 2224) in which the core rotation shaft (21) is rotatably fitted,
**characterized in that**
the needle disc roller being maintained so as to be movable forward and backward in the roller guiding holder (14);
the roller bracket (22) being installed to be rectilinearly movable forward and backward along a guide groove (142), wherein each of the pair of shaft mounting walls (222) is slidably fitted in the guide groove (142);
the needle disc roller further comprising a drug delivery mechanism for delivering a drug in a drug container to ring-shaped needle discs (26) of the needle disc roller (20) in response to the movement of the needle disc roller (20) back and forth as the needle disc roller (20) is pressed against the skin.

2. The needle disc roller apparatus of claim 1, wherein the roller bobbin (24) comprises:
a hollow rotation shaft (241) rotatably installed on the outer circumferential surface of the core rotation shaft (21) so as to be rotatable about the core rotation shaft (21), and;
a pair of bobbin covers (242a and 242b) provided at both ends of the hollow rotation shaft (241) to prevent separation of the plurality of ring-shaped needle discs (26) and the plurality of ring-shaped spacers (28).

3. The needle disc roller apparatus of claim 2, wherein at least one bobbin cover (242b) of the pair of bobbin covers (242a and 242b) is detachably assembled with respect to the hollow rotation shaft (241).

4. The needle disc roller apparatus of claim 2, wherein at least one key groove (2412) is formed on the outer circumference of the hollow rotation shaft (241) in the longitudinal direction, each of the plurality of ring-shaped needle discs (26) comprises a first key (265) on an inner surface thereof to be fitted in the key groove (2412), each of the plurality of ring-shaped spacers comprises a second key on an inner circumferential surface thereof to be fitted in the key groove (2412), and the plurality of ring-shaped needle discs (26) and the plurality of ring-shaped spacers (28) are restrained by the hollow rotation shaft (241) by fitting the first key (265) and the second key (285) of the plurality of ring-shaped needle discs (26) and the plurality of ring-shaped spacers (28) into the key groove (2412) in a row.

5. The needle disc roller apparatus of claim 1, **characterized in that** the drug delivery mechanism comprises:
a valve membrane (123) of a viscoelastic material having a valve hole (1232) which is formed in the connection cap (12) and communicates with a front end opening of the drug container; and
a valve operating part (23) selectively opening and closing the valve hole (1232) by moving forward and backward together with the needle disc roller (20), and
the valve operating part (23) comprises:
a rod portion (231) moving forward and backward integrally with the needle disc roller (20) while being inserted into the valve hole (1232) without a gap;
a tapered pressing portion (232) formed in the middle of the rod portion (231) and elastically expanding the valve hole (1232) by being fitted into the valve hole (1232) when moving backward together with the rod portion (231); and
at least one drug passage incision groove (233) formed on the tapered pressing portion (232) and connecting to the valve hole (1232) to open the valve hole (1232) when the valve hole (1232) is expanded by the tapered pressing portion (232).

6. The needle disc roller apparatus of claim 1 **characterized in that**
the connection cap (12) is made of viscoelastic material, and is coupled to cover a front end opening of the drug container (2);
**characterized in that** the drug delivery mechanism comprises:
a valve membrane (123) of a viscoelastic material having a valve hole (1232) which is formed in the viscoelastic connection cap (12) and communicates with a front end opening of the drug container; and
a valve operating part (23) selectively opening and closing the valve hole (1232) by moving forward and backward together with the needle disc roller (20), and
the valve operating part (23) comprises:
a rod portion (231) moving forward and backward integrally with the needle disc roller (20) while being inserted into the valve hole (1232) without a gap;
a tapered pressing portion (232) formed in the middle of the rod portion (231) and elastically expanding the valve hole (1232) by being fitted into the valve hole (1232) when moving backward together with the rod portion (231); and
at least one drug passage incision groove (233) formed on the tapered pressing portion (232) and connecting to the valve hole (1232) to open the valve hole (1232) when the valve hole (1232) is expanded by the tapered pressing portion (232).

7. The needle disc roller apparatus of claim 6, wherein the viscoelastic connection cap (12) comprises:
a rear outer tube (120a) closely adhered to the outer circumferential surface of the front end opening of the drug container (2) when being coupled to the front end of the drug container (2);
a rear inner tube (120b) inserted into an opening and closely adhered to the inner circumferential surface of the opening thereof;
a plate-shaped ring (120c) connecting the inner circumference of the front end of the rear outer tube (120a) and the outer circumference of the front end of the rear inner tube (120b);
a front outer tube (121) closely adhered to the outer surface of a portion of a rear end of the roller guiding holder (14) when being coupled to the rear end of the roller guiding holder (14); and
a front inner tube (128) having a concentric axis and formed inside the front outer tube (121).

8. The needle disc roller apparatus of claim 7, wherein the valve membrane (123) is integrally formed inside the hollow of the front inner tube (128), and the valve hole (1232) is formed at the center of the valve membrane (123).

9. The needle disc roller apparatus of claim 6, wherein the tapered pressing portion (232) has a shape gradually increasing in diameter from the rear end having the same diameter as the rod portion (231) toward the front end having a diameter larger than the diameter of the rod portion (231).

10. The needle disc roller apparatus of claim 9, wherein the drug passage incision groove (233) forms a pair with the drug passage incision groove (233) formed at one side of the tapered pressing portion (232) while being formed at the other side of the tapered pressing portion (232), and the pair of drug passage incision grooves (233 and 233) are formed adjacent to the outer circumference of the needle disc (26) and opposite to each other.

11. The needle disc roller apparatus of claim 1,
**characterized in that** the connection cap (12) is made of viscoelastic material, and is coupled to cover a front end opening of the drug container (2);
and **characterized in that** the Lin connection cap (12) comprises:
a rear outer tube (120a) having a circular inner circumference that is closely adhered to the outer circumferential surface of the front end opening of the drug container (2) when being coupled to the front end of the drug container (2);
a rear inner tube (120b) having a circular outer circumference that is inserted into the opening of the drug container (2) and is closely adhered to the inner circumferential surface of the opening thereof;
a plate-shaped ring (120c) connecting the inner circumference of the front end of the rear outer tube (120a) and the outer circumference of the front end of the rear inner tube (120b);
a front outer tube (121) closely adhered to the outer surface of a rear end step (140) of the roller guiding holder (14) when being coupled to the rear end of the roller guiding holder (14);
a front inner tube (128) having a concentric axis and formed inside the front outer tube (121);
a hook (122) formed so as to direct to the inside from the rear end of the rear outer tube (120a) and thus hooked at a stopping protrusion (2a) of the drug container (2) in a one-touch manner when being assembled with the viscoelastic connection cap (12) and the drug container (2); and
a ring-shaped retaining cover (121a) extending inward from the front end of the front outer casing (121) and elastically deformed such that a rear end step (140) of the roller guiding holder (14) is inserted into the inside thereof.

12. The needle disc roller apparatus of claim 11, wherein a valve membrane (123) is integrally formed inside the hollow of the front inner tube part (128), the valve membrane (123) has a valve hole (1232) at the center thereof, and the valve hole (1232) is elastically expanded or contracted by an external force.

13. The needle disc roller apparatus of claim 12, wherein the drug delivery mechanism comprises a valve operating part (23) provided on the needle disc roller (20) together with the valve membrane (123), and the valve operating part (23) interacts with the valve membrane (123) in accordance with the forward and backward movement of the valve disc (12) to open or close the valve hole (1232).

14. The needle disc roller apparatus of claim 13, wherein the valve operating part comprises:
a rod portion moving forward and backward integrally with the needle disc roller (20) while being fitted in the valve hole without a gap;
a tapered pressing portion (232) formed at the center of the rod portion (231) and having a shape gradually increasing in diameter from the rear end having the same diameter as the rod portion (231) toward the front end having a diameter larger than the diameter of the rod portion (231); and
a drug passage incision groove (233) formed in the tapered pressing portion (232), wherein when the rod portion (231) moves backward and the tapered pressing portion (232) presses the valve membrane (123), the valve membrane (123) is pushed by the tapered pressing portion (232) to expand the valve hole (1232), and the valve hole (1232) is connected to the drug passage incision groove (233) and is opened while being expanded by the tapered pressing portion (232).

## Patentansprüche

1. Nadelscheibenrollenvorrichtung mit:
einer Verbindungskappe (12),
einem Rollenführungshalter (14), der mit einem vorderen Ende der Verbindungskappe (12) gekoppelt ist, und
einer Nadelscheibenrolle (20),
wobei die Nadelscheibenrolle (20) aufweist:
einen Rollenhalter (22);
eine Kerndrehwelle (21), die drehbar in den Rollenbügel (22) eingebaut ist, eine Rollenspule (24), die mit der Kerndrehwelle (21) drehbar eingebaut ist, und
mehrere ringförmige Nadelscheiben (26) und mehrere ringförmige Abstandhalter (28), die sämtlich durch die Rollenspule (24) gehalten sind und zusammen mit der Rollenspule (24) um die Kerndrehwelle (21) drehen,
wobei der Rollenbügel (22) aufweist:
einen Basisbereich (221); und
zwei Wellenbefestigungswände (222 und 222), die vertikal auf beiden Seiten der Vorderfläche des Basisbereichs ausgebildet sind, und
wobei die beiden Wellenbefestigungswände (222) Wellenlöcher (2224 und 2224) aufweisen, in welche die Kerndrehwelle (21) drehbar eingesetzt ist, **dadurch gekennzeichnet,**
**dass** die Nadelscheibenrolle vorwärts und rückwärts bewegbar in dem Rollenführungshalter (14) gehalten ist;
**dass** der Rollenbügel (22) entlang einer Führungsnut (142) geradlinig vorwärts und rückwärts bewegbar eingebaut ist, wobei jede der beiden Wellenbefestigungswände (222) gleitend bewegbar in die Führungsnut (142) eingesetzt ist;
**dass** die Nadelscheibenrolle ferner einen Medikamentenabgabemechanismus aufweist, welcher in Reaktion auf das Vor- und Zurückbewegen der Nadelscheibenrolle (20) ein in einem Medikamentenbehälter befindliche Medikament an die ringförmigen Nadelscheiben (26) der Nadelscheibenrolle (20) abgibt, während die Nadelscheibenrolle (20) gegen die Haut gedrückt wird.

2. Nadelscheibenrollenvorrichtung nach Anspruch 1, bei welcher die Rollenspule (24) aufweist:
eine hohle Drehwelle (241), die auf der Außenumfangsfläche der Kerndrehwelle (21) derart drehbar angebracht ist, dass sie um die Kerndrehwelle (21) drehbar ist; und
zwei Spulenabdeckungen (242a und 242b), die an beiden Enden der hohlen Drehwelle (241) vorgesehen sind, um ein Lösen der mehreren ringförmigen Nadelscheiben (26) und der mehreren ringförmigen Abstandhalter (28) zu verhindern.

3. Nadelscheibenrollenvorrichtung nach Anspruch 2, bei welcher mindestens eine Spulenabdeckung (242b) der beiden Spulenabdeckung (242a und 242b) in Bezug auf die hohle Drehwelle (241) lösbar montiert ist.

4. Nadelscheibenrollenvorrichtung nach Anspruch 2, bei welcher mindestens eine Passfedernut (2412) in Längsrichtung in der Außenumfangsfläche der hohlen Drehwelle (241) ausgebildet ist, jede der mehreren ringförmigen Nadelscheiben (26) eine erste Passfeder (265) auf einer Innenfläche derselben zum Einsetzen in die Passfedernut (2412) aufweist, jeder der mehreren ringförmigen Abstandhalter eine zweite Passfeder auf einer Innenumfangsfläche desselben zum Einsetzen in die Passfedernut (2412) aufweist, und die mehreren ringförmigen Nadelscheiben (26) und die mehreren ringförmigen Abstandhalter (28) durch die hohle Drehwelle (241) durch Einsetzen der ersten Passfeder (265) und der zweiten Passfeder (285) der mehreren ringförmigen Nadelscheiben (26) und der mehreren ringförmigen Abstandhalter (28) in einer Reihe in die Passfedernut (2412) zurückgehalten sind.

5. Nadelscheibenrollenvorrichtung nach Anspruch 1, **dadurch gekennzeichnet, dass** der Medikamentenabgabemechanismus aufweist:
eine Ventilmembran (123) aus einem viskoelastischen Material mit einem Ventilloch (1232), das in der Verbindungskappe (12) ausgebildet ist und mit einer Vorderendöffnung des Medikamentenbehälters verbunden ist; und
ein Ventilbetätigungsteil (23), welches das Ventilloch (1232) wahlweise öffnet und schließt, indem es sich zusammen mit der Nadelscheibenrolle (20) vorwärts und rückwärts bewegt; und
wobei das Ventilbetätigungsteil (23) aufweist:
einen Stabbereich (231), der sich einstückig mit der Nadelscheibenrolle (20) vorwärts und rückwärts bewegt, wobei er spaltfrei in den Ventilloch (1232) eingesetzt ist;
einen sich verjüngenden Pressbereich (232), der in der Mitte des Stabbereichs (231) ausgebildet ist und das Ventilloch (1232) elastisch aufweitet, indem er in das Ventilloch (1232) eingeführt wird, während er sich zusammen mit dem Stabbereich (231) zurückbewegt; und
mindestens eine Medikamentendurchlasseinschnittrille (233), die in dem sich verjüngenden Pressbereich (232) gebildet ist und mit dem Ventilloch (1232) verbunden ist, um das Ventilloch (1232) zu öffnen, wenn das Ventilloch (1232) durch den sich verjüngenden Pressbereich (232) aufgeweitet ist.

6. Nadelscheibenrollenvorrichtung nach Anspruch 1, **dadurch gekennzeichnet,**
**dass** die Verbindungskappe (12) aus viskoelastischem Material besteht und zum Abdecken einer Vorderendöffnung des Medikamentenbehälters (2) verbunden ist,
**dadurch gekennzeichnet, dass** der Medikamentenabgabemechanismus aufweist:
eine Ventilmembran (123) aus einem viskoelastischen Material mit einem Ventilloch (1232), das in der viskoelastischen Verbindungskappe (12) ausgebildet ist und mit einer Vorderendöffnung des Medikamentenbehälters verbunden ist; und
ein Ventilbetätigungsteil (23), welches das Ventilloch (1232) wahlweise öffnet und schließt, indem es sich zusammen mit der Nadelscheibenrolle (20) vorwärts und rückwärts bewegt; und
wobei das Ventilbetätigungsteil (23) aufweist:
einen Stabbereich (231), der sich einstückig mit der Nadelscheibenrolle (20) vorwärts und rückwärts bewegt, wobei er spaltfrei in den Ventilloch (1232) eingeführt wird;
einen sich verjüngenden Pressbereich (232), der in der Mitte des Stabbereichs (231) ausgebildet ist und das Ventilloch (1232) elastisch aufweitet, indem er in das Ventilloch (1232) eingeführt wird, während er sich zusammen mit dem Stabbereich (231) zurückbewegt; und
mindestens eine Medikamentendurchlasseinschnittrille (233), die in dem sich verjüngenden Pressbereich (232) gebildet ist und mit dem Ventilloch (1232) verbunden ist, um das Ventilloch (1232) zu öffnen, wenn das Ventilloch (1232) durch den sich verjüngenden Pressbereich (232) aufgeweitet ist.

7. Nadelscheibenrollenvorrichtung nach Anspruch 6, bei welcher die viskoelastische Verbindungskappe (12) aufweist:
ein hinteres äußeres Rohr (120a), das eng an der Außenumfangsfläche der Vorderendöffnung des Medikamentenbehälters (2) anliegt, wenn es mit dem Vorderende des Medikamentenbehälters (2) gekoppelt ist;
ein hinteres inneres Rohr (120b), das in eine Öffnung eingesetzt ist und eng an der Innenumfangsfläche der Öffnung anliegt;
einen plattenförmigen Ring (120c), der den innenumfang des Vorderendes des hinteren äußeren Rohrs (120a) und den Außenumfang der Vorderendes des hinteren inneren Rohrs (120b) verbindet;
ein vorderes äußeres Rohr (121), das eng an der Außenfläche eines Bereichs eines hinteren Endes des Rollenführungshalters anliegt, wenn es mit dem Rollenführungshalter (14) verbunden ist; und
ein vorderes inneres Rohr (128), das eine konzentrische Achse aufweist und in dem vorderen äußeren Rohr (121) ausgebildet ist.

8. Nadelscheibenrollenvorrichtung nach Anspruch 7, bei welcher die Ventilmembran (123) einstückig in dem Hohlraum des vorderen inneren Rohrs (128) ausgebildet ist, und das Ventilloch (1232) in der Mitte der Ventilmembran (123) gebildet ist.

9. Nadelscheibenrollenvorrichtung nach Anspruch 6, bei welcher der sich verjüngende Pressbereich (232) eine Form aufweist, die von dem hinteren Ende, das denselben Durchmesser aufweist wie der Stabbereich (231) zum vorderen Ende (231), das einen Durchmesser aufweist, der größer als der Durchmesser des Stabbereichs (231) ist, allmählich zunimmt.

10. Nadelscheibenrollenvorrichtung nach Anspruch 9, bei welcher die Medikamentendurchlasseinschnittrille (233) ein Paar mit der auf einer Seite des sich verjüngenden Drückbereichs (232) gebildeten Medikamentendurchlasseinschnittrille (233) bildet, wobei sie auf der anderen Seite des sich verjüngenden Drückbereichs (232) gebildet ist, und die beiden Medikamentendurchlasseinschnittrillen (233 und 233) nahe dem Außenumfang der Nadelscheibe (26) und einander gegenüberliegend gebildet sind.

11. Nadelscheibenrollenvorrichtung nach Anspruch 1, **dadurch gekennzeichnet,**
**dass** die Verbindungskappe (12) aus viskoelastischem Material besteht und zum Abdecken einer Vorderendöffnung des Medikamentenbehälters (2) verbunden ist; und
**dadurch gekennzeichnet, dass** die Verbindungskappe (12) aufweist:
ein hinteres äußeres Rohr (120a) mit einem kreisförmigen Innenumfang, das eng an der Außenumfangsfläche der Vorderendöffnung des Medikamentenbehälters (2) anliegt, wenn es mit dem Vorderende des Medikamentenbehälters (2) gekoppelt ist;
ein hinteres inneres Rohr (120b) mit einem kreisförmigen Außenumfang, das in die Öffnung des Medikamentenbehälters (2) eingesetzt ist und eng an der Innenumfangsfläche der Öffnung desselben anliegt;
einen plattenförmigen Ring (120c), der den innenumfang des Vorderendes des hinteren äußeren Rohrs (120a) und den Außenumfang der Vorderendes des hinteren inneren Rohrs (120b) verbindet;
ein vorderes äußeres Rohr (121), das eng an der Außenfläche einer hinteren Endstufe (140) des Rollenführungshalters (14) anliegt, wenn es mit dem hinteren Ende des Rollenführungshalters (14) verbunden ist; und
ein vorderes inneres Rohr (128), das eine konzentrische Achse aufweist und in dem vorderen äußeren Rohr (121) ausgebildet ist;
ein Haken (122), der derart ausgebildet ist, dass er von dem inneren Ende des hinteren äußeren Rohrs (120a) nach innen gerichtet ist, und somit auf One-Touch-Weise mit einem Stoppvorsprung (2a) des Medikamentenbehälters (2) verhakt wird, wenn er mit der viskoelastischen Verbindungskappe (12) und dem Medikamentenbehälter (2) zusammengesetzt wird; und
eine ringförmige Rückhalteabdeckung (121a), die sich von dem vorderen Ende des vorderen äußeren Gehäuses (121) nach innen erstreckt und derart elastisch verformt ist, dass eine hintere Endstufe (140) des Rollenführungshalters (14) in das Innere derselben eingesetzt ist.

12. Nadelscheibenrollenvorrichtung nach Anspruch 11, bei welcher eine Ventilmembran (123) einstückig in dem Hohlraum des vorderen inneren Rohrteils (128) gebildet ist, die Ventilmembran (123) ein Ventilloch (1223) in deren Mitte aufweist, und das Ventilloch (1232) durch eine externe Kraft elastisch aufgeweitet oder zusammengezogen wird.

13. Nadelscheibenrollenvorrichtung nach Anspruch 12, bei welcher der Medikamentenausgabemechanismus ein Ventilöffnungsteil (23) aufweist, das zusammen mit der Ventilmembran (123) an der Nadelscheibenroll (20) vorgesehen ist, und das Ventilöffnungsteil (23) mit der Ventilmembran (123) gemäß der Vorwärts- und Rückwärtsbewegung der Ventilscheibe (12) zusammenwirkt, um das Ventilloch (1232) zu öffnen oder zu schließen.

14. Nadelscheibenrollenvorrichtung nach Anspruch 13, bei welcher das Ventilbetätigungsteil aufweist:
einen Stabbereich, der sich mit der Nadelscheibenrolle (20) vorwärts und rückwärts bewegt, wobei er spaltfrei in den Ventilloch (1232) eingesetzt ist;
einen sich verjüngenden Pressbereich (232), der in der Mitte des Stabbereichs (231) ausgebildet ist und eine Form aufweist, die von dem hinteren Ende, das denselben Durchmesser aufweist wie der Stabbereich (231) zum vorderen Ende (231), das einen Durchmesser aufweist, der größer als der Durchmesser des Stabbereichs (231) ist, allmählich zunimmt; und
eine in dem sich verjüngenden Drückbereich (232) ausgebildete Medikamentendurchlasseinschnittrille (233),
wobei, wenn sich der Stabbereich (231) rückwärts bewegt und der sich verjüngende Drückbereich (232) gegen die Ventilmembran (123) drückt, die Ventilmembran (123) von dem sich verjüngenden Drückbereich (232) gedrückt wird, um das Ventilloch (1232) aufzuweiten, und das Ventilloch (1232) mit der Medikamentendurchlasseinschnittrille (233) verbunden wird und geöffnet wird, während es durch den sich verjüngenden Drückbereich (232) aufgeweitet ist.

## Revendications

1. Rouleau à disques à aiguilles, comprenant :
un capuchon de liaison (12) ;
un support de guidage de rouleau (14) accouplé à une extrémité avant du capuchon de liaison (12) ; et
un rouleau à disques à aiguilles (20),
dans lequel le rouleau à disques à aiguilles (20) comprend :
un support pour rouleau (22) ;
un arbre de rotation de noyau (21) installé de manière rotative pour le support pour rouleau (22), une bobine pour rouleau (24) installée de manière rotative pour l'arbre de rotation de noyau (21) ; et
une pluralité de disques à aiguilles (26) en forme d'anneau et une pluralité d'espaceurs en forme d'anneau (28) qui sont tous retenus par la bobine pour rouleau (24) et tournent autour de l'arbre de rotation de noyau (21) conjointement avec la bobine pour rouleau (24),
le support pour rouleau (22) comprend :
une partie base (221) ; et
une paire de parois de montage d'arbre (222 et 222) formées verticalement de part et d'autre de la surface avant de la partie base (221), et
la paire de parois de montage d'arbre (222)
ayant des orifices pour arbre (2224 et 2224) dans lesquels l'arbre de rotation de noyau (21) est posé de manière rotative,
**caractérisé en ce que**
le rouleau à disques à aiguilles est maintenu de manière à pouvoir se déplacer vers l'avant et vers l'arrière dans le support de guidage de rouleau (14) ;
le support pour rouleau (22) étant installé de manière à pouvoir se déplacer de façon rectiligne vers l'avant et vers l'arrière le long d'une rainure de guidage (142), dans lequel chacune des parois de la paire de parois de montage d'arbre (222) est posée de manière coulissante dans la rainure de guidage (142) ;
le rouleau à disques à aiguilles comprenant en outre un mécanisme d'administration de médicament pour l'administration d'un médicament dans un contenant de médicament aux disques à aiguilles (26) en forme d'anneau du rouleau à disques à aiguilles (20) en réponse au mouvement de va-et-vient du rouleau à disques à aiguilles (20) tandis que le rouleau à disques à aiguilles (20) est pressé contre la peau.

2. Appareil à rouleau à disques à aiguilles selon la revendication 1, dans lequel la bobine pour rouleau (24) comprend :
un arbre de rotation creux (241) installé de manière rotative sur la surface circonférentielle extérieure de l'arbre de rotation de noyau (21) de manière à pouvoir tourner autour de l'arbre de rotation de noyau (21), et ;
une paire de couvercles de bobine (242a et 242b) fournis aux deux extrémités de l'arbre de rotation creux (241) pour empêcher une séparation de la pluralité de disques à aiguilles (26) en forme d'anneau et de la pluralité d'espaceurs en forme d'anneau (28).

3. Appareil à rouleau à disques à aiguilles selon la revendication 2, dans lequel au moins un couvercle de bobine (242b) de la paire de couvercles de bobine (242a et 242b) est assemblé de manière amovible par rapport à l'arbre de rotation creux (241).

4. Appareil à rouleau à disques à aiguilles selon la revendication 2, dans lequel au moins une rainure de clavette (2412) est formée sur la circonférence extérieure de l'arbre de rotation creux (241) dans la direction longitudinale, chaque disque de la pluralité de disques à aiguilles (26) en forme d'anneau comprend une première clavette (265) sur une surface intérieure de celui-ci destinée à être posée dans la rainure de clavette (2412), chaque espaceur de la pluralité d'espaceurs en forme d'anneau comprend une seconde clavette sur une surface circonférentielle intérieure de celui-ci destinée être posée dans la rainure de clavette (2412), et la pluralité de disques à aiguilles (26) en forme d'anneau et la pluralité d'espaceurs en forme d'anneau (28) sont retenues par l'arbre de rotation creux (241) en posant la première clavette (265) et la seconde clavette (285) de la pluralité de disques à aiguilles (26) en forme d'anneau et de la pluralité d'espaceurs en forme d'anneau (28) consécutivement dans la rainure de clavette (2412).

5. Appareil à rouleau à disques à aiguilles selon la revendication 1, **caractérisé en ce que** le mécanisme d'administration de médicament comprend :
une membrane de soupape (123) constituée d'un matériau viscoélastique ayant un orifice de soupape (1232) qui est formé dans le capuchon de liaison (12) et communique avec une ouverture d'extrémité avant du contenant de médicament ; et
une pièce de commande de soupape (23) ouvrant et fermant sélectivement l'orifice de soupape (1232) en se déplaçant vers l'avant et vers l'arrière conjointement avec le rouleau à disques à aiguilles (20), et
la pièce de commande de soupape (23) comprend :
une partie tige (231) se déplaçant vers l'avant et vers l'arrière intégralement avec le rouleau à disques à aiguilles (20) tout en étant insérée dans l'orifice de soupape (1232) sans espace ;
une partie pression conique (232) formée au milieu de la partie tige (231) et qui élargit élastiquement l'orifice de soupape (1232) en s'insérant dans l'orifice de soupape (1232) lorsqu'elle se déplace vers l'arrière conjointement avec la partie tige (231) ; et
au moins une rainure d'incision de passage de médicament (233) formée sur la partie pression conique (232) et reliée à l'orifice de soupape (1232) pour ouvrir l'orifice de soupape (1232) lorsque l'orifice de soupape (1232) est élargi par la partie pression conique (232).

6. Appareil à rouleau à disques à aiguilles selon la revendication 1, **caractérisé en ce que**
le capuchon de liaison (12) est constitué d'un matériau viscoélastique et est accouplé pour couvrir une ouverture d'extrémité avant du contenant de médicament (2) ;
**caractérisé en ce que** le mécanisme d'administration de médicament comprend :
une membrane de soupape (123) constituée d'un matériau viscoélastique ayant un orifice de soupape (1232) qui est formé dans le capuchon de liaison (12) viscoélastique et communique avec une ouverture d'extrémité avant du contenant de médicament ; et
une pièce de commande de soupape (23) ouvrant et fermant sélectivement l'orifice de soupape (1232) en se déplaçant vers l'avant et vers l'arrière conjointement avec le rouleau à disques à aiguilles (20), et
la pièce de commande de soupape (23) comprend :
une partie tige (231) se déplaçant vers l'avant et vers l'arrière intégralement avec le rouleau à disques à aiguilles (20) tout en étant insérée dans l'orifice de soupape (1232) sans espace ;
une partie pression conique (232) formée au milieu de la partie tige (231) et qui élargit élastiquement l'orifice de soupape (1232) en s'insérant dans l'orifice de soupape (1232) lorsqu'elle se déplace vers l'arrière conjointement avec la partie tige (231) ; et
au moins une rainure d'incision de passage de médicament (233) formée sur la partie pression conique (232) et reliée à l'orifice de soupape (1232) pour ouvrir l'orifice de soupape (1232) lorsque l'orifice de soupape (1232) est élargi par la partie pression conique (232).

7. Appareil à rouleau à disques à aiguilles selon la revendication 6, dans lequel le capuchon de liaison (12) viscoélastique comprend :
un tube extérieur arrière (120a) étroitement collé à la surface circonférentielle extérieure de l'ouverture d'extrémité avant du contenant de médicament (2) lorsqu'il est accouplé à l'extrémité avant du contenant de médicament (2) ;
un tube intérieur arrière (120b) inséré dans une ouverture et étroitement collé à la surface circonférentielle intérieure de l'ouverture de celle-ci ;
un anneau en forme de plaque (120c) reliant la circonférence intérieure de l'extrémité avant du tube extérieur arrière (120a) et la circonférence extérieure de l'extrémité avant du tube intérieur arrière (120b) ;
un tube extérieur avant (121) étroitement collé à la surface extérieure d'une partie d'une extrémité arrière du support de guidage de rouleau (14) lorsqu'il est accouplé à l'extrémité arrière du support de guidage de rouleau (14) ; et
un tube intérieur avant (128) ayant un axe concentrique et étant formé à l'intérieur du tube extérieur avant (121).

8. Appareil à rouleau à disques à aiguilles selon la revendication 7, dans lequel la membrane de soupape (123) est intégralement formée à l'intérieur du creux du tube intérieur avant (128), et l'orifice de soupape (1232) est formé au centre de la membrane de soupape (123).

9. Appareil à rouleau à disques à aiguilles selon la revendication 6, dans lequel la partie pression conique (232) a une forme augmentant progressivement en diamètre à partir de l'extrémité arrière ayant le même diamètre que la partie tige (231) vers l'extrémité avant ayant un diamètre plus grand que le diamètre de la partie tige (231).

10. Appareil à rouleau à disques à aiguilles selon la revendication 9, dans lequel la rainure d'incision de passage de médicament (233) forme une paire avec la rainure d'incision de passage de médicament (233) formée au niveau d'un côté de la partie pression conique (232) tout en étant formée au niveau de l'autre côté de la partie pression conique (232), et la paire de rainures d'incision de passage de médicament (233 et 233) sont formées de manière à être adjacentes à la circonférence extérieure du disque à aiguilles (26) et opposées l'une à l'autre.

11. Appareil à rouleau à disques à aiguilles selon la revendication 1,
**caractérisé en ce que** le capuchon de liaison (12) est constitué d'un matériau viscoélastique et est accouplé pour couvrir une ouverture d'extrémité avant du contenant de médicament (2) ;
et **caractérisé en ce que** le capuchon de liaison (12) comprend :
un tube extérieur arrière (120a) ayant une circonférence intérieure circulaire qui est étroitement collée à la surface circonférentielle extérieure de l'ouverture d'extrémité avant du contenant de médicament (2) lorsqu'elle est accouplée à l'extrémité avant du contenant de médicament (2) ;
un tube intérieur arrière (120b) ayant une circonférence extérieure circulaire qui est insérée dans l'ouverture du contenant de médicament (2) et est étroitement collée à la surface circonférentielle intérieure de l'ouverture de celui-ci ;
un anneau en forme de plaque (120c) reliant la circonférence intérieure de l'extrémité avant du tube extérieur arrière (120a) et la circonférence extérieure de l'extrémité avant du tube intérieur arrière (120b) ;
un tube extérieur avant (121) étroitement collé à la surface extérieure d'un taquet d'extrémité arrière (140) du support de guidage de rouleau (14) lorsqu'il est accouplé à l'extrémité arrière du support de guidage de rouleau (14) ;
un tube intérieur avant (128) ayant un axe concentrique et étant formé à l'intérieur du tube extérieur avant (121) ;
un crochet (122) formé de manière à se diriger vers l'intérieur à partir de l'extrémité arrière du tube extérieur arrière (120a) et ainsi accroché au niveau d'une saillie d'arrêt (2a) du contenant de médicament (2) d'une manière à une touche lorsqu'il est assemblé avec le capuchon de liaison (12) viscoélastique et le contenant de médicament (2) ; et
un couvercle de retenue en forme d'anneau (121a) s'étendant vers l'intérieur à partir de l'extrémité avant du boîtier extérieur avant (121) et déformé élastiquement de telle sorte qu'un taquet d'extrémité arrière (140) du support de guidage de rouleau (14) est inséré à l'intérieur de celui-ci.

12. Appareil à rouleau à disques à aiguilles selon la revendication 11, dans lequel une membrane de soupape (123) est intégralement formée à l'intérieur du creux de la partie tube intérieur avant (128), la membrane de soupape (123) a un orifice de soupape (1232) au centre de celle-ci, et l'orifice de soupape (1232) est élastiquement élargi ou contracté par une force externe.

13. Appareil à rouleau à disques à aiguilles selon la revendication 12, dans lequel le mécanisme d'administration de médicament comprend une pièce de commande de soupape (23) fournie sur le rouleau à disques à aiguilles (20) conjointement avec la membrane de soupape (123), et la pièce de commande de soupape (23) interagit avec la membrane de soupape (123) selon le mouvement vers l'avant et vers l'arrière du disque de soupape (12) pour ouvrir ou fermer l'orifice de soupape (1232).

14. Appareil à rouleau à disques à aiguilles selon la revendication 13, dans lequel la pièce de commande de soupape comprend :
une partie tige se déplaçant vers l'avant et vers l'arrière intégralement avec le rouleau à disques à aiguilles (20) tout en étant posée dans l'orifice de soupape sans espace ;
une partie pression conique (232)
formée au centre de la partie tige (231) et
ayant une forme augmentant progressivement en diamètre à partir de l'extrémité arrière ayant le même diamètre que la partie tige (231) vers l'extrémité avant ayant un diamètre plus grand que le diamètre de la partie tige (231) ; et
une rainure d'incision de passage de médicament (233) formée dans la partie pression conique (232),
dans lequel, lorsque la partie tige (231) se déplace vers l'arrière et que la partie pression conique (232) exerce une pression sur la membrane de soupape (123), la membrane de soupape (123) subit une pression exercée par la partie pression conique (232) pour élargir l'orifice de soupape (1232), et l'orifice de soupape (1232) est relié à la rainure d'incision de passage de médicament (233) et est ouvert tout en étant élargi par la partie pression conique (232).
